Europäisches Patentamt

European Patent Office

Office européen des brevets

(11) Publication number: **0 406 511 A1**

# EUROPEAN PATENT APPLICATION

(21) Application number: 90100047.1

(22) Date of filing: 02.01.90

(51) Int. Cl.⁵: **C12N 15/51**, A61K 39/29, G01N 33/576

(30) Priority: 28.06.89 JP 163715/89

(43) Date of publication of application:
09.01.91 Bulletin 91/02

(84) Designated Contracting States:
AT BE CH DE DK ES FR GB GR IT LI LU NL SE

(71) Applicant: SANWA KAGAKU KENKYUSHO CO., LTD.
No. 35, Higashi-sotobori-cho
Higashi-ku Nagoya-shi Aichi-ken(JP)

(72) Inventor: Hattori, Nobu
3-32, Izumigaoka 1-chome
Kanazawa-shi Ishikawa-ken(JP)
Inventor: Murakami, Seishi
12-13 wa, Okuwa-machi
Kanazawa-shi Ishikawa-ken(JP)
Inventor: Inagaki, Yutaka
30-12, Kodatsuno 1-chome
Kanazawa-shi Ishikawa-ken(JP)
Inventor: Yanagi, Masayuki
Kasamai Sanhaitsu 2FB, 6-10 Kasamai
1-chome
Kanazawa-shi Ishikawa-ken(JP)
Inventor: Sawai, Kiichi
35, Higashi-sotobori-cho
Higashi-sotobori-cho
Higashi-ku Nagoya Aichi-ken(JP)

(74) Representative: Wächtershäuser, Günter, Dr.
Tal 29
D-8000 München 2(DE)

(54) Cloning of non A non B hepatitis virus gene and expression thereof.

(57) A single-stranded DNA encoding a part of non A non B hepatitis virus gene, a double-stranded DNA consisting of the single-stranded DNA and its complementary single-stranded DNA, a process for the preparation thereof and a cloned vector for expression thereof to produce an antinogeous material due to the virus.

## CLONING OF NON A NON B HEPATITIS VIRUS GENE AND EXPRESSION THEREOF

The present invention relates to a single-stranded DNA encoding a part of non A non B (hereinafter referred to as -NANB-) hepatitis virus gene, a double-stranded DNA consisting of the single-stranded DNA and its complementary single-stranded DNA, a process for the preparation thereof, and a cloned vector for expression thereof to produce an antigenous material due to the virus.

NANB hepatitis occupies about 90 - 95% of hepatitis diseases due to the blood transfusion and a positive remedy thereto has not yet been established. Therefore, it has been strongly demanded to develop a diagnostic agent, an agent for treating blood for transfusion to make the possible NANB hepatitis virus harmless, and a protective vaccine therefor.

As a result of researches on antigen of NANB hepatitis virus in recent years, it has been reported that a single-stranded RNA encoding 10000 nucleotides may concern thereto [Jap. Pat. Nos. Sho 62 - 249999(A) and 63 - 91328(A)]. In recently issued news papers, further, a report by members belonging to National Institute of Health, Japan to the effect that NANB hepatitis virus was isolated and another report by members belonging to Okayama Medical College to the effect that a gene of the virus was isolated have been informed. However, it has not been confirmed whether these are sure NANB hepatitis virus and sure structural gene thereof, or not.

An object of the invention is to bring a possibility on diagnosis and cure of NANB hepatitis as well as for developing a treatment of blood for transfusion and a protective vaccine for NANB hepatitis, by obtaining at least a part of NANB hepatitis virus with use of so-called "Techniques of Biotechnology" and by making same to its clone.

The inventors have carefully and energetically studied and investigated on NANB hepatitis virus to finally obtain at least a part of DNA fragments thereof and succeed a cloning thereof, so that a structure of the fragment has been determined to attain the object.

A single-stranded DNA according to the invention comprises about 840 nucleotides which encodes a part of amino acid sequence for NANB hepatitis virus gene.

A double-stranded DNA according to the invention consists of the single-stranded DNA and its complementary single stranded DNA.

The single-stranded DNA has the following nucleotide sequence.

```
5'--- TGATGGAACAGTTGAAATGAATGCTCATATACAAAAGCTAACCTTAACGT
           10        20        30        40        50


     GAAGTGGAGTTAACTTGCAGCCAACACCATTGCATTACAAACCCTAACAC
           60        70        80        90       100


     ACACACATAATGAGAAAGTAGGCTTATTACACCTGGCGATTATCTCGCCT
          110       120       130       140       150


     TTTATCTACCATCTTTGAATAATGTAACTTATAAACACGTCGTTTTAAAG
          160       170       180       190       200


     CGCTGTGGCCGTCCTGAAAAGATGTGTCAATAAATGAATTTAATCCATAG
          210       220       230       240       250


     AATCCACGTTGACTTCACTACTTATTAAGATATTGTAACAAATCACGGAA
          260       270       280       290       300
```

```
GTGTGGAATATTTGTTATGGCTTTTAATTAAACACTAAACACAATACAAT
        310       320       330       340       350

GTCAAAATGGCACGGGCTTTATGCCCTGCTACAAGTTATCACGAACAGAC
        360       370       380       390       400

TGTGCTACCGTCACACACCTGTATAAACTCCGTCCCAACACAGAACAACG
        410       420       430       440       450

ACATGCAATTAGAACAGTAAGGAACATTGTTAAAGAACATCGTTGACATG
        460       470       480       490       ·500

TATATATGTCTATGCGCTTTGACATGTATATATGTCTATGCGCTGCATCA
        510       520       530       540       550

CATCGAGAAAATTATGTGCCCGCCGCACGGCATTATGGGGCGACTATGCC
        560       570       580       590       600

AAGTTAAATATGGAATGTTCAATGCCTTATCGCGCTGACTTGTCCCAGCC
        610       620       630       640       650

CTACCCCAGCACCTAATCCACACAACCAGACAACTTCTGCACAAACTGAA
        660       670       680       690       700

GTTACTGGTAAGGTGTTTCTTTTTAGTTTTTGTGTGTTTCTGCAAGTTTG
        710       720       730       740       750

GTTTTTGTATAAAATGTGTGTAAGAGTGTCCCTTGGCTTCTCTCTGGCCC
        760       770       780       790       800

CCTCACTCATGTGGAATCCCAAGATGGGGCCAGTGAA --- 3'
        810       820       830
```

wherein A, C, G and T are a deoxyribonucleotide having adenine, cytosine, guanine or thymine base.

According to the invention, the double-stranded DNA can be obtained by extracting and purifying RNA from a particulate fraction of blood plasma obtained from a patient with NANB hepatitis, preparing a double-stranded DNA fragment with use of the RNA, as a template, ligating EcoRI linker to the double-stranded DNA fragment, and then digesting the DNA fragment with EcoRI. The single-stranded DNA can be obtained by separating the double-stranded DNA fragment in a manner known per se , for instance by treating the double-stranded DNA fragment at 90°C for about 3 minutes and then ice cooling the same to cause a separation thereof.

A separation of the particulate fraction from blood plasma can be carried out by solubilize with use of

3

TEN buffer [50mM Toris chloride buffer (pH 8.0), 2mM EDTA, 150nM NaCl, and 1mM PMSF], precipitating with use of polyethylene glycol solution, obtaining a precipitation fraction through a high speed centrifugal treatment, solubilizing the precipitation fraction with TEN buffer to remove insoluble fraction through a high speed centrifugal treatment, overlying the resulting solution on a cushion of sucrose solution in TEN to obtain a precipitation fraction through a ultracentrifugation, repeating one or more times the last-mentioned operations, solubilizing the resulting precipitation fraction with use of TEN buffer, adding GIT in the resulting solution, and overlying the solution on a cushion of CsCl in TEN to obtain a precipitation fraction through a ultracentrifugation. The extraction of RNA from the precipitation fraction can be carried out with use of an organic solvent. The purification of the extracted RNA can be carried out by making the same into an aqueous solution, adding glycogen as a carrier and then causing precipitation by adding ethanol.

The preparation of said double-stranded DNA from the purified RNA can be carried out in a manner known per se , for instance, by incuvating the RNA in the presence of a primer and a reverse transcriptase to carry out the first strand synthesis (in this case, a double-stranded fragment shall be prepared, which fragment consists of the RNA strand and its complementary single-stranded DNA), incuvating the resulting double-stranded fragment in the presence of a ribonuclease and a DNA polymerase to carry out the second strand synthesis (in this case, a double-stranded DNA fragment shall be prepared, which consists of a single-stranded DNA separated from the double-stranded DNA fragment in the first strand synthesis and its complementary single-stranded DNA), treating the resulting double-stranded DNA fragment with a $T_4$ DNA polymerase to give blunt ends thereto, extracting the DNA fragment having the blunt ends with use of an organic solvent, and digesting the same with use of EcoRI. As the primer, a random hexamer, oligo(dt) or the like can be employed. As the organic solvent for extracting RNA and the fragment with blunt ends, chloroform, a mixture of chloroform and phenol, or the like may be listed.

The resulting double-stranded DNA can be made into its clone with use of techniques known per se , for instance by inserting the DNA into vectors and screening the vectors. As a cloning therefor, it is convenient to select an in vitro packaging method, when a pharge is selected as the vector, but a colony-hybridization method is preferable, when a plasmid is selected as the vector. The screening can be carried out by a southern hybridization method between the resulting clones as a probe and a host DNA and selecting one having no homology with the host DNA, as a desired recombinant vector with an insert of said double-stranded DNA.

At the present time, it has not yet been confirmed whether the double-stranded DNA in question is sure gene of NANB hepatitis virus or not, namely the cloned vector has been derived from NANB hepatitis virus or not, but can be estimated that the double-stranded DNA relates to one of characteristic parts of structural gene of NANB hepatitis virus, in view of said fact that the cloned vector does not hybridize with the host DNA.

As the vector, λ gt10, M13mp18, M13mp19 or the like pharge as well as pU118, pU119 or the like plasmid can be exemplified. These are known expression vectors, so that an antigenous material due to NANB hepatitis virus can be produced in large amount, by inserting the cloned vector into Escherichia coli, yeast or the like microorganism or an animal cell and cultivating the same, so that the antigenous product can be supplied for various research organizations to elucidate characteristics, pharmacological action and others thereof. It may possible to develop a vaccine for NANB hepatitis, by reducing a toxicity of the antinogeous material with use of conventional techniques such as a passage immunization method. If the antigenous material shall be administered to an animal for immunologically producing an antibody to obtain the same, a medicine for curing NANB hepatitis and an agent for treating trasfusional blood may be obtained. The medicine and agent can be stably supplied, by making the antibody into its mono- or polyclone. Further, if a dilution chart shall be prepared with use of the antibody, a diagnosis of a carrier of NANB hepatitis, or an extent of progress or recovery of hepatitis, hepatotumor due to NANB hepatitis virus can be made possible.

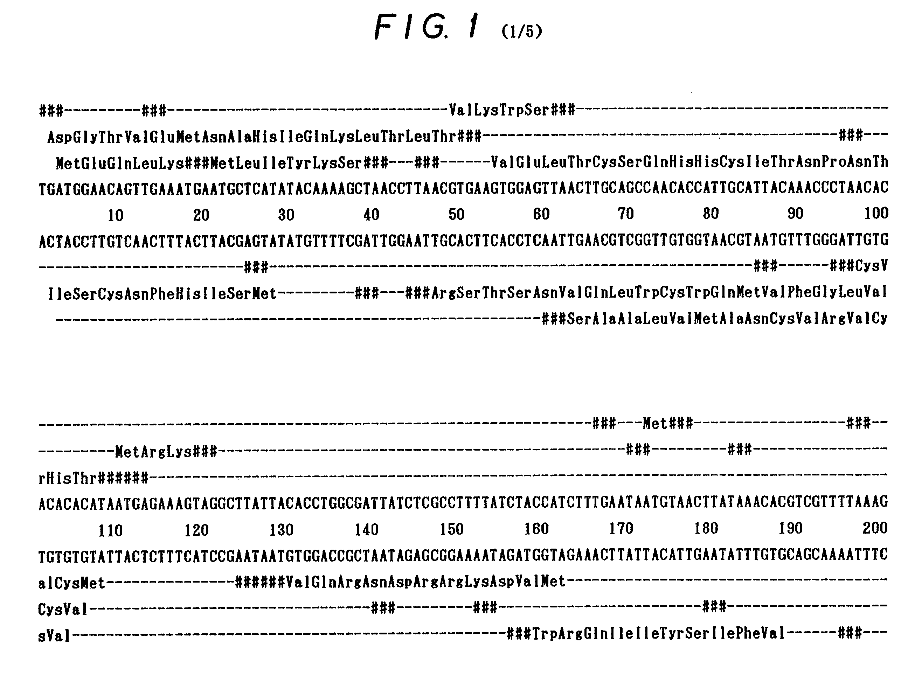

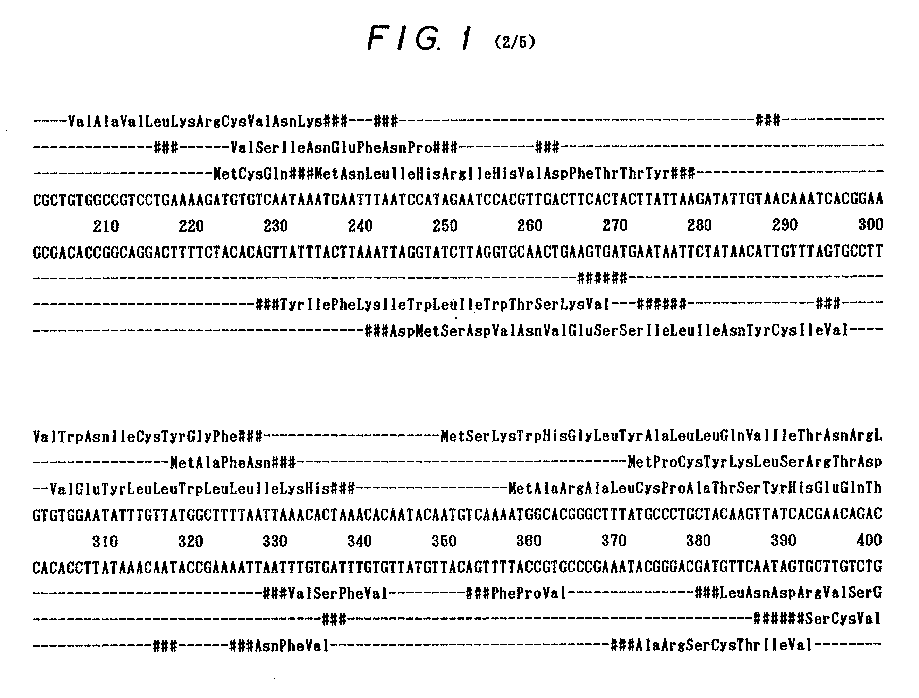

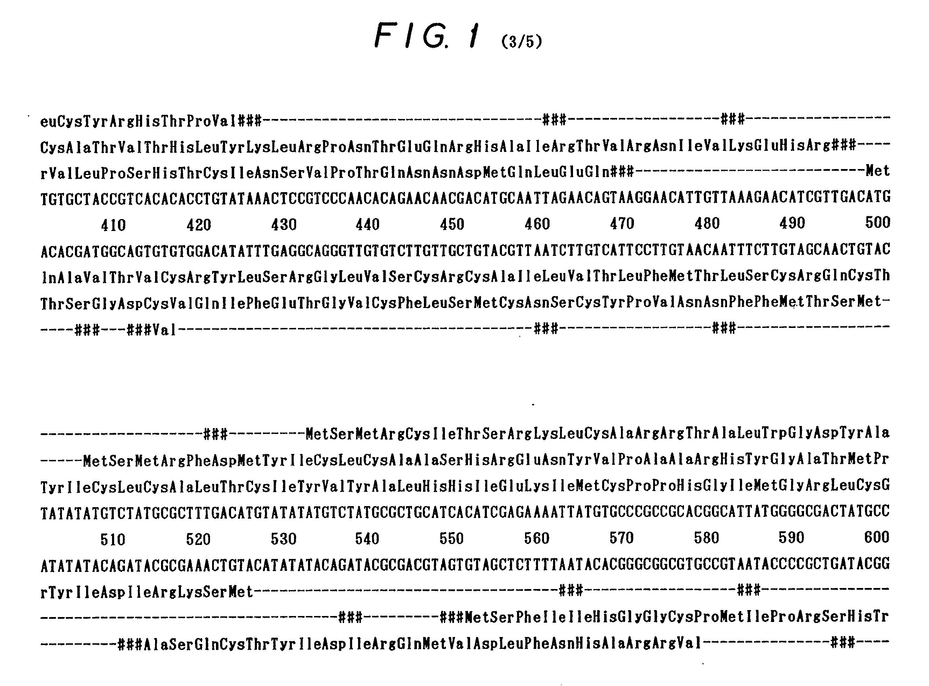

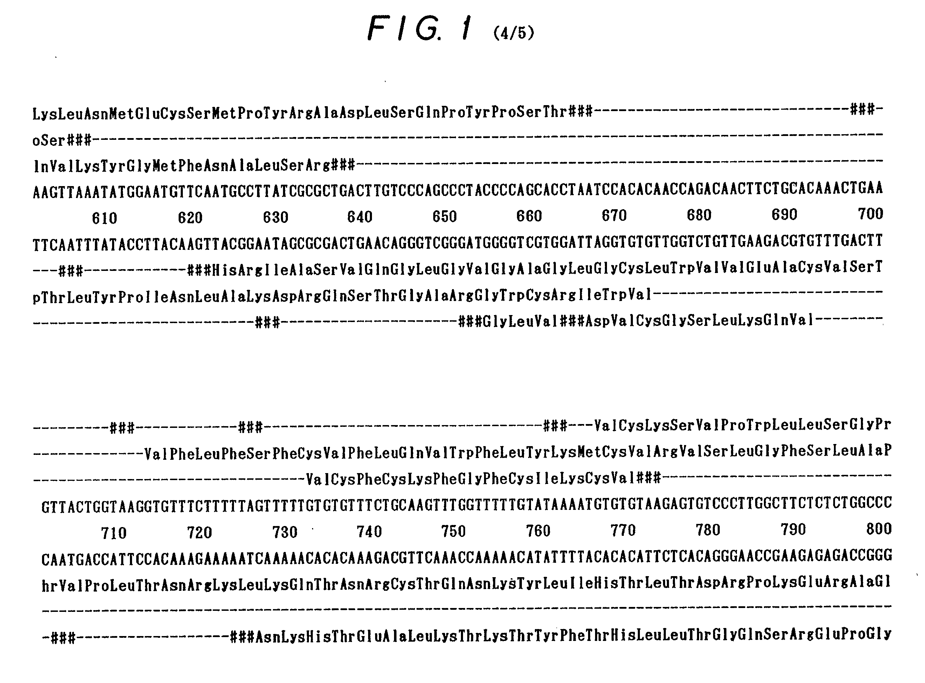

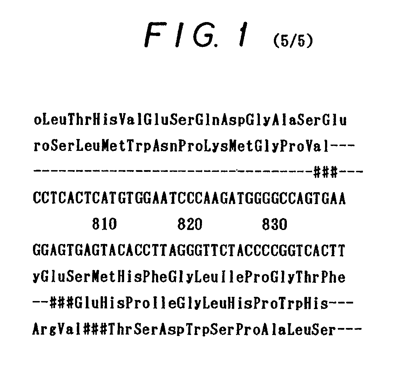

The invention will now be further explained with reference to Examples and a Test Example which shall refer to the single drawing of Fig. 1 showing a nucleotide sequence determined on a DNA fragment of the invention and amino acid sequences estimated from the nucleotide sequence.

Example 1

a) Isolation and purification of poly(a)RNA comprising mRNA of NANB hepatitis virus

To a blood plasma obtained from a patient with NANB hepatitis, 20% polyethylene glycol in TEN (TEN buffer) was added, and the resulting solution was stirred, left to stand for about 30 minutes, and then high-speed centrifugalized to obtain a precipitation fraction.

The precipitation fraction was solubilized by adding the same into TEN of 5-folds in volume, and high-speed centrifugalized to remove an insoluble fraction.

The resulting solution was overlaid on cushions of 15% and 60% sucrose solution in TEN, and ultracentrifugalized for 12 hours with use of a rotor (Type : SW-28) at 25000rpm to obtain a precipitation fraction. After repeated the operations, resulting particulate fraction was overlaid on a cushion of 15% sucrose solution in TEN, ultracentrifugalized for 12 hours with use of the SW-28 rotor at 25000rpm to obtain a precipitation fraction which was dissolved in TEN.

To the solution, GIT was added, and the resulting solution was overlaid on a cushion of CsCl in TEN (density : 1.7), ultracentrifugalized for 12 hours with use of the SW-28 rotor at 35000rpm to obtain a precipitation fraction which shall be referred to as "RNA fraction".

Two staged extraction was carried out on the RNA fraction, namely in the first place with use of a mixture of chloroform and phenol, and in the second place with use of chloroform. To a separated aqueous phase, glycogen as a carrier was add (final concentration : $20\mu$ g/ml), adding ethanol to cause a precipitation, and centrifugalized to obtain a precipitation fraction which was referred to as "purified poly(A)-RNA".

b) Separation from mRNA from purified poly(A)RNA and preparation of double-stranded DNA fragment

The purified poly(A)RNA obtained in said Item (a) and oligo(dT) as a primer [a random hexamer may be employed in lieu of oligo(dT)] were incuvated at 42°C for 45 minutes, in the presence of a reverse transcriptase (20 units), to carry out a first strand synthesis (The operation provides a double-stranded fragment consisting of the single-stranded RNA as a template and its complementary single-stranded DNA).

To the double-stranded fragment, RNase H (0.8 units) and DNA polymerase (23 units) were added to incuvate the same at 12°C for 60 minutes, 22°C for 60 minutes, and 70°C for 10 minutes, to carry out a second strand synthesis (The operations cause a separation of constitutional single-stranded fragments from the double-stranded fragment obtained by said first strand synthesis, and provide a desired double-stranded DNA fragment consisting of the separated single-stranded DNA and its complementary single-stranded DNA).

The resulting double-stranded DNA was treated by adding $T_4$ DNA polymerase (2.0 units) and incuvating at 37°C for 10 minutes, so that the DNA fragment has now blunt ends.

To the blunt ends of the DNA fragment extracted with use of phenol and chloroform, EcoRI linker was ligated with use of $T_4$ ligase, digested with use of EcoRI and purified to obtain a desired double-stranded DNA fragment.

The double-strand DNA fragment can be separated into constitutional single-stranded DNA fragments, by treating the same with use of conventional techniques.

Test Example

A nucleotide sequence of the double-stranded DNA fragment was checked and determined with use of a method known per se (for instance, Maxam-Gilbert method and others). Results of the determination are shown in Fig. 1, and the DNA fragment had 837 base pairs.

In the Figure, the 4th line shows a nucleotide sequence of one of the strands, for the DNA fragment prepared with use of the mRNA as the template, the 5th line is number of nucleotides in the sequence commencing from thymine (T) [this shall also be designated as "Uracil (U)"], the 6th line shows a nucleotide sequence of the other single-stranded DNA complemental to the aforesaid strand shown in the 4th line, as well as the 1st to 3rd lines and the 4th to 9th lines show an amino acid sequence to be estimated from the nucleotide sequences given in the 4th and 6th lines, wherein a symbol "###" in the amino acid sequences shows a position to be estimated as --termination codon-- from the nucleotide sequences.

Example 2

5

a) Insertion of DNA fragment into vector

As a vector, a pharge of λ gt10 was selected and cut with use of EcoRI to obtain a λ gt10 arm.

The DNA fragment obtained in Example 1 and having EcoRI recognition site in each end and the λ gt10 arm as obtained above were ligated with use of T₄ DNA ligase and the pharges were screened by in vitro packaging method to obtain clones with an insert of the DNA, as cloned pharge vector.

b) Selection of clone

The clones obtained by the operations described in said Item (a) were employed as a probe and subjected to southern hybridization to select a clone among them, which has no homology with a host DNA.

**Claims**

1. A single-stranded DNA comprising about 840 nucleotides which encodes a part of an amino acid sequence for non A non B hepatitis virus gene, or a double-stranded DNA consisting of said single-stranded DNA and its complementary single-stranded DNA.

2. A single-stranded DNA or double-stranded DNA as claimed in Claim 1, wherein said single-stranded DNA has the nucleotide sequence of

```
5'--- TGATGGAACAGTTGAAATGAATGCTCATATACAAAAGCTAACCTTAACGT
            10        20        30        40        50


      GAAGTGGAGTTAACTTGCAGCCAACACCATTGCATTACAAACCCTAACAC
            60        70        80        90        100


      ACACACATAATGAGAAAGTAGGCTTATTACACCTGGCGATTATCTCGCCT
            110       120       130       140       150


      TTTATCTACCATCTTTGAATAATGTAACTTATAAACACGTCGTTTTAAAG
            160       170       180       190       200


      CGCTGTGGCCGTCCTGAAAAGATGTGTCAATAAATGAATTTAATCCATAG
            210       220       230       240       250


      AATCCACGTTGACTTCACTACTTATTAAGATATTGTAACAAATCACGGAA
            260       270       280       290       300


      GTGTGGAATATTTGTTATGGCTTTTAATTAAACACTAAACACAATACAAT
            310       320       330       340       350


      GTCAAAATGGCACGGGCTTTATGCCCTGCTACAAGTTATCACGAACAGAC
            360       370       380       390       400
```

6

```
TGTGCTACCGTCACACACCTGTATAAACTCCGTCCCAACACAGAACAACG
      410       420       430       440       450

ACATGCAATTAGAACAGTAAGGAACATTGTTAAAGAACATCGTTGACATG
      460       470       480       490       500

TATATATGTCTATGCGCTTTGACATGTATATATGTCTATGCGCTGCATCA
      510       520       530       540       550

CATCGAGAAAATTATGTGCCCGCCGCACGGCATTATGGGGCGACTATGCC
      560       570       580       590       600

AAGTTAAATATGGAATGTTCAATGCCTTATCGCGCTGACTTGTCCCAGCC
      610       620       630       640       650

CTACCCCAGCACCTAATCCACACAACCAGACAACTTCTGCACAAACTGAA
      660       670       680       690       700

GTTACTGGTAAGGTGTTTCTTTTTAGTTTTTGTGTGTTTCTGCAAGTTTG
      710       720       730       740       750

GTTTTTGTATAAAATGTGTGTAAGAGTGTCCCTTGGCTTCTCTCTGGCCC
      760       770       780       790       800

CCTCACTCATGTGGAATCCCAAGATGGGGCCAGTGAA --- 3'
      810       820       830
```

wherein A, C, G and T are a deoxyribonucleotide having adenine, cytosine, guanine or thyimine base.

3. A process for the preparation of a single-stranded DNA comprising about 840 nucleotides which encodes a part of an amino acid sequence for non A non B hepatitis virus gene, or a double-stranded DNA consisting of said single-stranded DNA and its com plementary single-stranded DNA, which comprises steps of extracting and purifying RNA from a particulate fraction of blood plasma obtained from a patient with non A non B hepatitis, preparing a double-stranded DNA fragment with use of said RNA, as a template, ligating EcoRI linker to the double-stranded DNA fragment, and then digesting the DNA fragment with EcoRI, and if necessary, separating the double-stranded DNA fragment into constitutional single-stranded DNA fragments.

4. A cloned expression vector which has an insert of double-stranded DNA fragment consisting of a single-stranded DNA comprising about 840 nucleotides which encodes a part of an amino acid sequence for non A non B hepatitis virus gene, and its complementary single-stranded DNA.

5. A process for the preparation of a cloned expression vector which has an insert of double-stranded DNA fragment consisting of a single-stranded DNA comprising about 840 nucleotides which encodes a part of an amino acid sequence for non A non B hepatitis virus gene, and its complementary single-stranded DNA, which process comprises steps of extracting and purifying RNA from a particulate fraction of blood plasma obtained from a patient with non A non B hepatitis, preparing a double-stranded DNA fragment with use of

7

said RNA, as a template, ligating EcoRI linker to the double-stranded DNA fragment, and digesting the DNA fragment with EcoRI, ligating the resulting shortened chain double-stranded DNA fragment with a fragment of a vector to reconstruct the vector, separating clones with an insert of said DNA fragment though a screening, and selecting through southern hybridization a clone having no homology with a host DNA, when said screened clones are employed as a probe.

# FIG. 1 (1/5)

```
###----------###--------------------------------ValLysTrpSer###--------------------------------------------
 AspGlyThrValGluMetAsnAlaHisIleGlnLysLeuThrLeuThr###--------------------------------------------###---
  MetGluGlnLeuLys###MetLeuIleTyrLysSer###---###------ValGluLeuThrCysSerGlnHisHisCysIleThrAsnProAsnTh
TGATGGAACAGTTGAAATGAATGCTCATATACAAAAGCTAACCTTAACGTGAAGTGGAGTTAACTTGCAGCCAACACCATTGCATTACAAACCCTAACAC
        10        20        30        40        50        60        70        80        90       100
ACTACCTTGTCAACTTTACTTACGAGTATATGTTTTCGATTGGAATTGCACTTCACCTCAATTGAACGTCGGTTGTGGTAACGTAATGTTTGGGATTGTG
--------------------------###-------------------------------------------------------###------###CysV
 IleSerCysAsnPheHisIleSerMet---------###---###ArgSerThrSerAsnValGlnLeuTrpCysTrpGlnMetValPheGlyLeuVal
    --------------------------------------------------------###SerAlaAlaLeuValMetAlaAsnCysValArgValCy


---------------------------------------------###---Met###------------------###--
---------MetArgLys###------------------------------------------###---------###-----------------
rHisThr#####----------------------------------------------------------------
ACACACATAATGAGAAAGTAGGCTTATTACACCTGGCGATTATCTCGCCTTTTATCTACCATCTTTGAATAATGTAACTTATAAACACGTCGTTTTAAAG
       110       120       130       140       150       160       170       180       190       200
TGTGTGTATTACTCTTTCATCCGAATAATGTGGACCGCTAATAGAGCGGAAAATAGATGGTAGAAACTTATTACATTGAATATTTGTGCAGCAAAATTTC
alCysMet---------------######ValGlnArgAsnAspArgArgLysAspValMet--------------------------------------
CysVal-----------------------------------###---------###---------------------###--------------------
sVal----------------------------------------------###TrpArgGlnIleIleIleTyrSerIlePheVal------###---
```

EP 0 406 511 A1

*FIG. 1* (2/5)

```
----ValAlaValLeuLysArgCysValAsnLys###---###-----------------------------------------------###-------------
--------------###------ValSerIleAsnGluPheAsnPro###--------###----------------------------------------
----------------------MetCysGln###MetAsnLeuIleHisArgIleHisValAspPheThrThrTyr###-----------------------
CGCTGTGGCCGTCCTGAAAAGATGTGTCAATAAATGAATTTAATCCATAGAATCCACGTTGACTTCACTACTTATTAAGATATTGTAACAAATCACGGAA
        210       220       230       240       250       260       270       280       290       300
GCGACACCGGCAGGACTTTTCTACACAGTTATTTACTTAAATTAGGTATCTTAGGTGCAACTGAAGTGATGAATAATTCTATAACATTGTTTAGTGCCTT
---------------------------------------------------#####-------------------------------
--------------------------###TyrIlePheLysIleTrpLeuIleTrpThrSerLysVal---#####----------------###-----
-----------------------------------###AspMetSerAspValAsnValGluSerSerIleLeuIleAsnTyrCysIleVal----
```

```
ValTrpAsnIleCysTyrGlyPhe###--------------------MetSerLysTrpHisGlyLeuTyrAlaLeuLeuGlnValIleThrAsnArgL
------------------MetAlaPheAsn###--------------------------------------MetProCysTyrLysLeuSerArgThrAsp
--ValGluTyrLeuLeuTrpLeuLeuIleLysHis###------------------MetAlaArgAlaLeuCysProAlaThrSerTyrHisGluGlnTh
GTGTGGAATATTTGTTATGGCTTTTAATTAAACACTAAACACAATACAATGTCAAAATGGCACGGGCTTTATGCCCTGCTACAAGTTATCACGAACAGAC
        310       320       330       340       350       360       370       380       390       400
CACACCTTATAAACAATACCGAAAATTAATTTGTGATTTGTGTTATGTTACAGTTTTACCGTGCCCGAAATACGGGACGATGTTCAATAGTGCTTGTCTG
------------------------###ValSerPheVal--------###PheProVal----------------###LeuAsnAspArgValSerG
----------------------------------###----------------------------------------#####SerCysVal
------------------###------###AsnPheVal-----------------------------------###AlaArgSerCysThrIleVal--------
```

EP 0 406 511 A1

# FIG. 1 (3/5)

euCysTyrArgHisThrProVal###-------------------------------------###-----------------------###-------------------
CysAlaThrValThrHisLeuTyrLysLeuArgProAsnThrGluGlnArgHisAlaIleArgThrValArgAsnIleValLysGluHisArg###-----
rValLeuProSerHisThrCysIleAsnSerValProThrGlnAsnAsnAspMetGlnLeuGluGln###--------------------------------Met
TGTGCTACCGTCACACACCTGTATAAACTCCGTCCCAACACAGAACAACGACATGCAATTAGAACAGTAAGGAACATTGTTAAAGAACATCGTTGACATG
      410      420      430      440      450      460      470      480      490      500
ACACGATGGCAGTGTGTGGACATATTTGAGGCAGGGTTGTGTCTTGTTGCTGTACGTTAATCTTGTCATTCCTTGTAACAATTTCTTGTAGCAACTGTAC
lnAlaValThrValCysArgTyrLeuSerArgGlyLeuValSerCysArgCysAlaIleLeuValThrLeuPheMetThrLeuSerCysArgGlnCysTh
ThrSerGlyAspCysValGlnIlePheGluThrGlyValCysPheLeuSerMetCysAsnSerCysTyrProValAsnAsnPhePheMetThrSerMet-
-----###---###Val-----------------------------------------###-----------------------###-------------------

-----------------------###----------MetSerMetArgCysIleThrSerArgLysLeuCysAlaArgArgThrAlaLeuTrpGlyAspTyrAla
-----MetSerMetArgPheAspMetTyrIleCysLeuCysAlaAlaSerHisArgGluAsnTyrValProAlaAlaArgHisTyrGlyAlaThrMetPr
TyrIleCysLeuCysAlaLeuThrCysIleTyrValTyrAlaLeuHisHisIleGluLysIleMetCysProProHisGlyIleMetGlyArgLeuCysG
TATATATGTCTATGCGCTTTGACATGTATATATGTCTATGCGCTGCATCACATCGAGAAAATTATGTGCCCGCCGCACGGCATTATGGGGCGACTATGCC
      510      520      530      540      550      560      570      580      590      600
ATATATACAGATACGCGAAACTGTACATATATACAGATACGCGACGTAGTGTAGCTCTTTTTAATACACGGGCGGCGTGCCGTAATACCCCGCTGATACGG
rTyrIleAspIleArgLysSerMet----------------------------------------###-----------------------###------------------
---------------------------------###---------###MetSerPheIleIleHisGlyGlyCysProMetIleProArgSerHisTr
----------###AlaSerGlnCysThrTyrIleAspIleArgGlnMetValAspLeuPheAsnHisAlaArgArgVal---------------###-----

# FIG. 1 (4/5)

LysLeuAsnMetGluCysSerMetProTyrArgAlaAspLeuSerGlnProTyrProSerThr###-----------------------------###-

oSer###--------------------------------------------------------------------------------------

InValLysTyrGlyMetPheAsnAlaLeuSerArg###-----------------------------------------------------

AAGTTAAATATGGAATGTTCAATGCCTTATCGCGCTGACTTGTCCCAGCCCTACCCCAGCACCTAATCCACACAACCAGACAACTTCTGCACAAACTGAA

610       620       630       640       650       660       670       680       690       700

TTCAATTTATACCTTACAAGTTACGGAATAGCGCGACTGAACAGGGTCGGGATGGGGTCGTGGATTAGGTGTGTTGGTCTGTTGAAGACGTGTTTGACTT

---###-------------###HisArgIleAlaSerValGlnGlyLeuGlyValGlyAlaGlyLeuGlyCysLeuTrpValValGluAlaCysValSerT

pThrLeuTyrProIleAsnLeuAlaLysAspArgGlnSerThrGlyAlaArgGlyTrpCysArgIleTrpVal----------------------------

----------------------###--------------------###GlyLeuVal###AspValCysGlySerLeuLysGlnVal--------

----------###-------------###-----------------------------------###---ValCysLysSerValProTrpLeuLeuSerGlyPr

--------------ValPheLeuPheSerPheCysValPheLeuGlnValTrpPheLeuTyrLysMetCysValArgValSerLeuGlyPheSerLeuAlaP

----------------------------ValCysPheCysLysPheGlyPheCysIleLysCysVal###--------------------------

GTTACTGGTAAGGTGTTTCTTTTTAGTTTTTGTGTGTTTCTGCAAGTTTGGTTTTTGTATAAAATGTGTGTAAGAGTGTCCCTTGGCTTCTCTCTGGCCC

710       720       730       740       750       760       770       780       790       800

CAATGACCATTCCACAAAGAAAAATCAAAAACACACAAAGACGTTCAAACCAAAAACATATTTTACACACATTCTCACAGGGAACCGAAGAGAGACCGGG

hrValProLeuThrAsnArgLysLeuLysGlnThrAsnArgCysThrGlnAsnLysTyrLeuIleHisThrLeuThrAspArgProLysGluArgAlaGl

-----------------------------------------------------------------------------------------------

-###-----------------###AsnLysHisThrGluAlaLeuLysThrLysThrTyrPheThrHisLeuLeuThrGlyGlnSerArgGluProGly

EP 0 406 511 A1

# F I G. 1 (5/5)

```
oLeuThrHisValGluSerGlnAspGlyAlaSerGlu
roSerLeuMetTrpAsnProLysMetGlyProVal---
--------------------------------###---
CCTCACTCATGTGGAATCCCAAGATGGGGCCAGTGAA
        810       820       830
GGAGTGAGTACACCTTAGGGTTCTACCCCGGTCACTT
yGluSerMetHisPheGlyLeuIleProGlyThrPhe
--###GluHisProIleGlyLeuHisProTrpHis---
ArgVal###ThrSerAspTrpSerProAlaLeuSer---
```

European
Patent Office

EUROPEAN SEARCH
REPORT

Application Number

EP 90 10 0047

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 318 216  (CHIRON CORP.) <br> * The whole document * <br> − − − | 1,3-5 | C 12 N 15/51 <br> A 61 K 39/29 <br> G 01 N 33/576 |
| A | EP-A-0 293 274  (MITSUBISHI CHEMICAL INDUSTRIES) <br> − − − | | |
| A | SCIENCE, vol. 244, no. 4902, 21st April 1989, pages 362-364, Washington, DC, US; G. KUO et al.: "An assay for circulating antibodies to a major etiologic virus of human non-A, non-B hepatitis" <br> − − − | | |
| A | SCIENCE, vol. 244, no. 4902, 21st April 1989, pages 359-362, Washington, DC, US; Q.-L. CHOO et al.: "Isolation of a cDNA clone derived from a blood-borne non-A, non-B viral hepatitis genome" <br> − − − − − | | |

TECHNICAL FIELDS
SEARCHED (Int. Cl.5)

C 12 N
A 61 K
G 01 N

The present search report has been drawn up for all claims

| Place of search | Date of completion of search | Examiner |
|---|---|---|
| The Hague | 26 October 90 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document